(19) Europäisches Patentamt
European Patent Office
Office européen des brevets

(11) Veröffentlichungsnummer : 0 331 167 B1

(12) # EUROPÄISCHE PATENTSCHRIFT

(45) Veröffentlichungstag der Patentschrift :
**22.07.92 Patentblatt 92/30**

(51) Int. Cl.[5] : **C07F 9/10**, C07C 305/00, C12Q 1/34

(21) Anmeldenummer : **89103660.0**

(22) Anmeldetag : **02.03.89**

(54) **Substrate für Phospholipasen.**

(30) Priorität : **04.03.88 DE 3807123**

(43) Veröffentlichungstag der Anmeldung :
**06.09.89 Patentblatt 89/36**

(45) Bekanntmachung des Hinweises auf die Patenterteilung :
**22.07.92 Patentblatt 92/30**

(84) Benannte Vertragsstaaten :
**AT BE CH DE ES FR GB GR IT LI LU NL SE**

(56) Entgegenhaltungen :
**EP-A- 0 070 433**
**EP-A- 0 225 129**
**FR-A- 2 517 310**
**US-A- 4 485 045**
**SYNTHESIS - JOURNAL OF SYNTHETIC ORGANIC CHEMISTRY, Nr. 1, Januar 1987, Seiten 60-62, New York, US; E. HASEGAWA et al.: "Aconvenient synthesis of mixed-acid glycerophosphocholines"**

(73) Patentinhaber : **BOEHRINGER MANNHEIM GMBH**
**Sandhofer Strasse 112-132**
**W-6800 Mannheim-Waldhof (DE)**

(72) Erfinder : **Junius, Martina, Dr. rer. nat.**
**Eichenstrasse 4**
**W-8139 Bernried (DE)**
Erfinder : **Neumann, Ulrich, Dr. rer. nat.**
**Starenweg 12**
**W-8120 Weilheim (DE)**
Erfinder : **von der Eltz, Herbert, Dr. rer. nat.**
**In der Au 21**
**W-8120 Weilheim (DE)**

(74) Vertreter : **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach86 08 20**
**W-8000 München 86 (DE)**

EP 0 331 167 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Phospholipasen (PL) katalysieren die Hydrolyse von Estergruppen der sn-3-Phosphoglyceride.

Die Substrate bilden in wässriger Lösung Micellen, und die Enzyme wirken an der Lipid-Wasser-Grenzfläche.

Es ist eine Reihe von Phospholipasen bekannt, welche von besonderem Interesse sind, vor allem PL C und PL $A_2$ (die am besten untersuchte PL des menschlichen Körpers). Bei bestimmten Erkrankungen, wie z.B. Pankreatitis, Infektionskrankheiten, Autoimmunerkrankungen, Allergien erhöht sich die PL $A_2$-Konzentration im Blut und anderen Körperflüssigkeiten. Die Bestimmung der PL $A_2$-Aktivität hat daher diagnostisch beträchtliche Bedeutung.

PL C, insbesondere auch Phosphatidyl-Inositol-spezifische PL C = PInase gewann in letzter Zeit zunehmend an Bedeutung. Das Regulatorsystem Phosphatidyl-Inosit wird von vielen Forschergruppen intensiv bearbeitet. Zudem spielt PL C auch eine Rolle bei der Ablösung membrangebundener Proteine.

PL $A_2$ ist auch in einigen Schlangengiften enthalten (Kobra, Klapperschlange), eine erhöhte Konzentration ist lebensgefährlich. PL $A_2$ zählt zu den Verdauungsenzymen und die Bestimmung spielt nicht nur in der Klin. Chemie, sondern auch in der Biochemie, pharmazeutischen Chemie und Lebensmittelchemie eine große Rolle (G.E.Hoffmann, Dt.Ges.f.Klin.Chem. e.V. - Mitteilungen 4, 196 (86)).

Es sind bereits mehrere Phospholipasen-Meßmethoden bekannt, wie z.B. die titrimetrische Bestimmung der bei der Esterspaltung freigesetzten Fettsäure (Figarella, Scand.J.Gastroent. 6, 133 (71)) oder die Messung der radioaktiv markierten freigesetzten Fettsäure (Shakir, Anal.Biochem. 114, 64 (81)). Diese Verfahren sind aber für Routinezwecke sehr aufwendig und störanfällig.

Weiter sind photometrische Bestimmungsmethoden bekannt:

a) Hendrickson J. Lipid Res. 24, 1532 (83),

b) Hoffmann, Dt.Ges.f.Klin.Chem., Mitteilungen 4, 201 (86)

zu a):

Sie beruht auf der Freisetzung einer -SH Gruppe und deren Bestimmung mit DTNB, erwies sich aber als zu unempfindlich.

zu b):

Die vollenzymatische Methode zur Bestimmung der Fettsäure nach Fa. Wako, Japan ist aufwendig und stark störanfällig (Vielzahl von Pipettierschritten).

NEFA-C-Test

Bekannt sind auch fluoreszierende Phospholipide als Substrate (Thuren, Clin.Chem. 31, 714 (85)). Allerdings ist die Methode stark störanfällig und nur wenige Labors sind mit Fluoreszenzmeßgeräten ausgestattet.

Aus 1007 Synthesis-Journal of Synthetic Organic Chemistry (1987), January, Nr. 1, New York, USA ist ein Verfahren zur Synthese von Glycerophosphocholinestern mit zwei unterschiedlichen Säuregruppen bekannt. Diese Verbindungen enthalten Styrolgruppen und sind daher polymerisierbar. Zu den Eigenschaften wird angegeben, daß sie Vesikel und stabile polymerisierte Mikrokapseln bei Fotopolymerisation bilden. Ein Hinweis auf eine Eignung als Phospholipasesubstrate findet sich dort nicht. Die Verbindungen sind aufgrund ihres Styrolrestes auch stets verschieden von den erfindungsgemäßen Phospholipasesubstraten.

Beschrieben sind auch immunologische Verfahren zur Bestimmung von PL A:
Radioimmunassay: Nishijima, J.Biochem. 94, 137 (83) und Fluoreszenzimmunoassay: Eskola, Clin.Chem. 29, 1777 (83), die zwar hinsichtlich Empfindlichkeit ausreichen, jedoch nicht zwischen der aktiven Phospholipase A und ihrer inaktiven Vorstufe unterscheiden können.

Für die Bestimmung der Phospholipase C sind nur wenige Methoden bekannt. So ist es bekannt, nach Spaltung des Substrats durch PL C mit Lipase als Hilfsenzym Glycerin freizusetzen und dann eine vollenzymatische Bestimmung des freigesetzten Glycerins durchzuführen (Wahlefeld in Bergmeyer: Methoden der enzymatischen Analyse, 3.Aufl., Bd.II, Verlag Chemie Weinheim 1974, S.1878). Diese Methode ist aber sehr störanfällig und zeitraubend.

Weiterhin ist die Bestimmung der PL C durch Einsatz radioaktiv markierter Substrate publiziert (Waku, J.Biochem. 72, 149 (72)). Diese Methode ist jedoch umständlich und unempfindlich.

Daher besteht Bedarf an einem Farbtest, der mit einer einfachen Apparatur durchgeführt und direkt visuell kontrolliert werden kann.

Der Erfindung liegt daher die Aufgabe zugrunde, ein Substrat und einen Farbtest zur Bestimmung der Phospholipase unter Verwendung dieses Substrats zu schaffen, der die Nachteile der bekannten Tests nicht

aufweist, genaue Ergebnisse liefert, einfach in der Handhabung ist, hohe Empfindlichkeit besitzt und nur eine geringe lag-Phase aufweist, so daß die Adaptation an die verschiedenen Analysenautomatensysteme unschwierig ist.

Gelöst wird diese Aufgabe erfindungsgemäß durch ein Phospholipasesubstrat der allgemeinen Formel

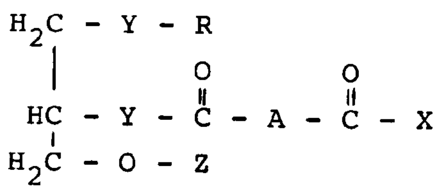

(I)

oder

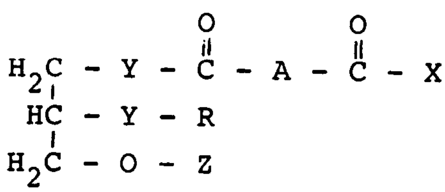

(II)

worin
A eine Alkylen- oder Alkenylengruppe mit 1 bis 16 C-Atomen,
R H oder eine Alkyl-, Alkenyl- oder Acylgruppe mit 1 bis 20 C-Atomen oder eine gegebenenfalls alkylsubstituierte Aryl- oder Aralkylgruppe mit 1 bis 8 C-Atomen in Alkylrest,
X den Rest einer aromatischen Hydroxy- oder Thiolverbindung und jedes Y unabhängig voneinander -S- oder -O-,
Z $-SO_3^{\ominus}$ oder

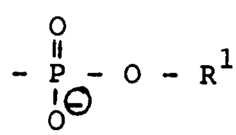

bedeuten, wobei $R^1$ sein kann ein Wasserstoffatom oder die Gruppe $(CH_2)_nNR^2_3$, in der n eine Zahl von 2 bis 4 und $R^2$ H oder $CH_3$ bedeuten,
oder Inositol,
oder Serin

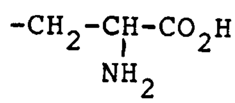

oder Glycerin.

Unter der Einwirkung der Phospholipase wird das erfindungsgemäße Phospholipasesubstrat gespalten unter Freisetzung der dem Rest X entsprechenden aromatischen Hydroxy- oder Thiolverbindung, die man entweder direkt optisch bestimmt oder mit einem geeigneten Chromophor oder Fluorophor kuppelt und das Kupplungsprodukt mißt oder eventuell nach Zugabe eines Hilfsenzyms mißt.

R weist vorzugsweise 6 bis 20 C-Atome, besonders bevorzugt 12 bis 18 C-Atome auf. Überraschenderweise erwiesen sich die Verbindungen mit R = Alkyl, Alkenyl oder Aralkyl als gute Phospholipasesubstrate, obwohl die natürlichen Substrate Acylgruppen tragen.

Beispiele für R sind Methyl-, Ethyl-, Propyl-, Butyl-, Pentyl-, Hexyl-, Heptyl-, Octyl-, Nonyl-, Decyl-, Undecyl, Dodecyl-, Tetradecyl-, Hexadecyl- und Octadecylreste als Alkylgruppen sowie die entsprechenden Acylgruppen wie die Acetyl-, Propionyl-, Butyryl-, Valeryl-, Capronyl-, Capryl-, Caprinyl-, Lauryl-, Myristyl-, Palmityl- und Stearylgruppe, die Oleyl-, Crotonyl-, Linolylgruppe, Phenyl-, Benzyl- oder Octylphenylgruppe.

Das erfindungsgemäße Phospholipasesubstrat enthält weiter den Rest einer Dicarbonsäure COOH-A-COOH, in der A vorzugsweise 3 bis 7 C-Atome aufweist. Beispiele für Säuren, von denen sich A ableitet, sind Malonsäure, Bernsteinsäure, Glutarsäure, Adipinsäure, Pimelinsäure, Korksäure, Azelainsäure, Sebacinsäure, Nonandicarbonsäure, Decandicarbonsäure und Undecandicarbonsäure. Die Säuren von Glutarsäure bis Azelainsäure sind bevorzugt. Für A mit mehr als 4 C-Atomen ist der Zusatz von einem Hilfsenzym empfehlenswert.

X kann eine aromatische Hydroxy- oder Thiolverbindung sein, die ein Chromophor darstellt oder erst durch eine Folgereaktion in einen Farbstoff überführt werden kann. Typische Beispiele sind Phenol, Thiophenol, Naphthol, Thionaphthol und deren Derivate sowie die an sich chromogenen Verbindungen wie der Resorufin-, Chlorphenolrot-, Indoxyl- oder Thiofluoreszeinrest. Eine erschöpfende Aufzählung der geeigneten Hydroxy- oder Thiolverbindungen ist aufgrund ihrer großen Zahl nicht möglich, die direkt chromophoren oder in Chromophore überführbaren aromatischen Hydroxy- oder Thiolverbindungen sind dem Fachmann jedoch bekannt.

Als Verbindungen der allgemeinen Formel (I) bzw. (II) werden
1-O-Octadecyl-2-glutarsäure-methylresorufinester-sn-glycero-3-phosphocholin,
1-O-Octadecyl-2-glutarsäure-p-nitrophenylester-sn-glycero-3-phosphocholin,
1-O-Dodecyl-2-glutarsäure-nitrophenylester-rac-glycero-3-sulfat,
1-O-Dodecyl-2-adipinsäure-nitrophenylester-rac-glycero-3-sulfat oder/und
1-O-Hexadecyl-2-glutarsäure-nitrophenylester-rac-glycero-3-sulfat bevorzugt.

Die genannte Folgereaktion zum Farbstoff kann entweder durch eine direkte Kupplung (z.B. mit einem Diazoniumsalz wie 4-Chlor-2-methylbenzoldiazoniumsalz (Fast Red), 4-Benzamido-2-methoxy-5-methylbenzoldiazoniumsalz (Fast Violet), diazotierte Sulfanilsäure, 2,4- und 2,5-substituierte Phenyldiazoniumsalze z.B. 2,4-Dichlorphenyldiazonium-1,5-naphthalin-disulfonsäure) erfolgen oder durch eine oxidative Kupplung z.B. mit 4-Aminoantipyrin oder anderen Aminopyrazolonen (wie Trimethylaminopyrazolon, Diaminoantipyrin) oder MBTHS (3-Methyl-2-benzothiazolinon-hydrazon-6-sulfonsäure).

Bevorzugt sind Chromophore, die eine geringe Polarität aufweisen und lipophil sind. Dabei sollte jedoch die Wasserlöslichkeit noch gewährleistet sein.

Der lipophile Charakter der o.g. Chromophore kann durch geeignete Substitution, wie z.B. mit Alkylgruppen, positiv beeinflußt werden. Als geeignete Substituenten für den Resorufinrest haben sich u.a. die Methyl-, Dimethyl- und Äthylgruppe sowie die Substitution mit Brom als geeignet erwiesen.

Die erfindungsgemäßen Verbindungen sind neu. Sie besitzen ein Asymmetriezentrum und sind daher optisch aktiv. Als Phospholipasesubstrat können sowohl die bei den üblichen Herstellungsmethoden anfallenden Racemate als auch die optischen Isomeren verwendet werden. Bevorzugt sind jedoch die optischen Isomeren.

Die Herstellung der erfindungsgemäßen Phospholipasesubstrate kann nach an sich bekannten Methoden erfolgen. So finden sich geeignete Verfahren zur Synthese der Ether-glycero- und Acyl-glycero-phosphocholine beispielsweise beschrieben in Methods in Enzymology, 98, 623 (1983) und Biochim.Biophys.Acta 666, 230 (81). Die Synthese von Sulfaten ist beispielsweise in Beilstein 2, EII, 356 beschrieben.

Ausgehend von beschriebenen 1-Alkyl- bzw. 1-Acyl-3-O-Trityl-glycerinverbindungen werden dann durch Umsetzung mit den entsprechenden Dicarbonsäureanhydriden in wasserfreiem Medium wie Chloroform/Pyridin die entsprechenden Glycerodicarbonsäure-Monoester erhalten. Geeignete Methoden zur Herstellung der Dicarbonsäureanhydride sind in Houben-Weyl-Müller "Methoden der organischen Chemie", Band IV/4, Seite 786 beschrieben.

Es kann anstelle der 3-O-Tritylgruppe auch eine andere Schutzgruppe verwendet werden, z.B. Benzyl-.

Die Veresterung des Monoesters mit der aromatischen Hydroxy- oder Thiolverbindung, von der sich der Rest X ableitet, kann beispielsweise durch direkte Umsetzung des Dicarbonsäuremonoesters mit dem aromatischen Alkohol oder Thiol in Gegenwart eines Wasser entziehenden Mittels wie Dicyclohexylcarbodiimid, durchgeführt werden. Alternativ wird der Dicarbonsäuremonoester zuerst in einen aktivierten Ester überführt, beispielsweise in den Hydroxysuccinimidester oder das Imidazolid und der aktivierte Ester wird dann mit dem aromatischen Alkohol oder Thiol umgesetzt.

Ebenso ist es auch möglich, zuerst einen Monoester der Dicarbonsäure mit dem aromatischen Alkohol oder Thiol herzustellen, beispielsweise den Adipinsäure-Mononitrophenylester oder Glutarsäuremonophenylester, und diesen dann mit dem Alkyl- bzw. -acylglycerin zu verestern, beispielsweise über intermediäre Bildung eines Säurechlorids, -anhydrids oder aktivierten Esters. Die Herstellung der Dicarbonsäuremonoester mit dem aromatischen Alkohol oder Thiol kann beispielsweise aus Säureanhydrid und aromatischer Verbindung im Molverhältnis 1:1 oder aus Dicarbonsäure und aromatischer Verbindung im Molverhältnis 2:1 oder aus einem

Dicarbonsäuremonoester mit leicht abspaltbarer Schutzgruppe und der aromatischen Verbindung erfolgen. Eine geeignete Methode ist beispielsweise in Arch. Pharm. 287, 514 (1954) beschrieben.

Alternativ kann der 1-O-Alkyl- bzw. 1-O-Acyl-3-O-Trityl-glycero-dicarbonsäure-monoester auch hergestellt werden, indem man zunächst einen Dicarbonsäuremonoester aus der Dicarbonsäure und einem leicht abspaltbaren Alkohol, wie z. B. Benzylalkohol oder 2,2,2-Trichlorethylalkohol herstellt und die so erhaltene Säure dann mit dem erwähnten Alkyl- bzw. Acyl-3-O-Trityl-Glycerin verestert. Anschließend wird die Schutzgruppe entfernt und die Umsetzung mit dem aromatischen Alkohol oder Thiol, wie oben beschrieben, vorgenommen.

Bei den oben beschriebenen Produkten sind dann Schutzgruppen abzuspalten und die C-3-OH-Position zu sulfatieren oder phosphorylieren.

Eine weitere Herstellmethode besteht darin, daß man zuerst ein geschütztes Glycerin wie 1,2-Isopropyliden-glycerin mit einem Dicarbonsäuremonoester umsetzt unter Bildung des entsprechenden geschützten Glycero-3-dicarbonsäure-diesters, dann die Schutzgruppe des Glycerins entfernt und die freigewordene 1-OH-Gruppe und die freigewordene 2-OH-Gruppe alkyliert bzw. acyliert. Schließlich wird die zuerst eingeführte Monoestergruppe (Carboxylschutzgruppe) abgespalten und mit dem aromatischen Alkohol oder Thiol umgesetzt. Nach der Spaltung der Schutzgruppe an C-1-OH wird phosphatiert oder sulfatiert.

Außerdem kann das gewünschte Substrat ausgehend von entsprechenden Lysoverbindungen (z.B. 1-Alkyl-glycero-3-sulfat) hergestellt werden, z.B. durch Umsetzung mit aktiviertem Dicarbonsäuremonoester.

Die oben beschriebene Herstellung der erfindungsgemäßen Substrate ist nicht erschöpfend und dem Fachmann stehen eine Reihe weiterer an sich bekannter Methoden zur Verfügung die es ihm ermöglichen, jede der erfindungsgemäßen Verbindungen ohne weiteres herzustellen. Aus den nach den oben erläuterten Verfahren erhaltenen racemischen Produkten können gewünschtenfalls die reinen optischen Isomeren nach bekannten Trennverfahren gewonnen werden. Ebenso lassen sich die Isomeren aber auch durch stereospezifisch ablaufende Synthese nach ebenfalls an sich bekannten Verfahren gewinnen.

Das erfindungsgemäße Verfahren zur optischen Bestimmung der Phospholipase ist dadurch gekennzeichnet, daß man ein erfindungsgemäßes Phospholipasesubstrat der Einwirkung der phospholipasehaltigen Probe unterwirft und die Menge der freigesetzten aromatischen Hydroxy- oder Thiolverbindung direkt oder nach Kopplung mit einem geeigneten Chromogen die daraus gebildete Farbe optisch bestimmt. Gegebenenfalls ist der Zusatz eines oder zweier Hilfsenzyme nötig.

Ein weiterer Gegenstand der Erfindung ist ein einfaches und gut haltbares Reagenz zur optischen Bestimmung der Phospholipase, welches neben einem erfindungsgemäßen Phospholipasesubstrat und Puffersubstanz auch ein oberflächenaktives Mittel, wie insbesondere Gallensäuresalz, ein Ca-Salz wie Calciumchlorid und gegebenenfalls einen chromogenen Kuppler oder/und ein Hilfsenzym enthält. Außerdem kann das Reagenz zweckmäßig auch Konservierungsmittel oder/und Aktivator enthalten.

In einer bevorzugten Zusammensetzung enthält dieses Reagenz

0,05 - 10 mg/ml Substrat, besonders bevorzugt 0,5 - 10 mg/ml Substrat,
2 - 50 mg/ml Gallensäure,
0,5 - 10 mM/l Calciumchlorid (Aktivator),
0 - 10 g/l Detergenz,
20 - 250 mM/l Puffersubstanz

jeweils bezogen auf gebrauchsfertige Lösung im Test.

Als oberflächenaktives Mittel der Gallensäuregruppe kommen Cholsäure, Taurocholsäure, Desoxycholsäure, Taurodesoxycholsäure, Glycodesoxycholsäure bzw. deren Alkalisalze, insbesondere das Natriumsalz, in Betracht. Die bevorzugte Menge beträgt 0,5 bis 1,5 mM/l. Alternativ oder zusätzlich kann das Reagenz auch ein oder mehrere nichtionische Detergentien enthalten.

Als Detergentien können sowohl ionische als auch nichtionische Detergentien verwendet werden. Vorzugsweise werden nichtionische Detergentien wie z.B. Triton [R] (Alkylarylpolyether) verwendet. Ein besonders geeigneter Konzentrationsbereich liegt zwischen 0 - 10 g/l. Besonders bevorzugt wird der Konzentrationsbereich zwischen 1 - 5 g/l verwendet.

Als Puffersubstanz eignen sich Puffer, welche in der Lage sind, im Rahmen des erfindungsgemäßen Reagenz einen pH-Wert zwischen 6,0 und 10,5 einzustellen. Der bevorzugte pH-Wertbereich liegt zwischen 7,0 und 9,5. Beispiele für geeignete Puffer sind Diethanolaminpuffer, Triethanolaminpuffer, Tris- Puffer und Goodpuffer, wie Hepes-Puffer (gut geeignet zum Zusatz vor der Lyophilisation), Taps-Puffer, CHES- Puffer (2-(Cyclohexylamino)ethansulfonsäure) und Bicine. Besonders bevorzugt wird Tris-Puffer. Die bevorzugte Puffersubstanzmenge liegt zwischen 20 und 250 mM/l.

Als Konservierungsmittel werden im Rahmen der Erfindung solche eingesetzt, die die enzymatische Aktivität der zu bestimmenden Phospholipase nicht beeinträchtigen. Besonders geeignet sind Alkaliazide, insbesondere Natriumazid. Auch andere Konservierungsmittel, wie z.B. Thiozid und andere schwefelhaltige Konservierungsmittel sind jedoch ebenfalls geeignet. Die bevorzugte Menge an Konservierungsmittel beträgt

0,001 bis 2 mg/ml.

Als Aktivatoren sind Erdalkaliionen, bevorzugt Calciumionen geeignet. Da diese mit Desoxycholsäure unlösliche Verbindungen eingehen, wird bei Gegenwart von Calcium als Gallensäure Taurodesoxycholsäure bevorzugt, da diese höhere Calciumkonzentrationen im Bereich von 1 bis 5 mMol zuläßt.

Wird das erfindungsgemäße Reagenz in trockener oder konzentrierter, zur Verdünnung auf die endgültige Zusammensetzung bestimmter Form eingesetzt, so enthält es die genannten Substanzen in entsprechenden Mengenverhältnissen, sowie vorzugsweise Schutzkolloid.

Als Schutzkolloid kommen die dem Chemiker hierfür bekannten Substanzen in Betracht, wie Polyhydroxyverbindungen, Serumalbumin, Polyvinylpyrrolidon, feste Polyethylenoxide und dergleichen. Bevorzugt werden Polyhydroxyverbindungen, insbesondere monomere oder polymere Pentose oder Hexose mit 1 bis 10 Pentose- bzw. Hexose-Einheiten im Molekül oder/und bei Zimmertemperatur fester Polyethylenglykol. Bevorzugte Beispiele von geeigneten Polyhydroxyverbindungen sind Mannit und ähnliche Zuckeralkohole, Oligosaccharid der Glucose, Mannose, Maltoheptaose, Polyethylenglykol mit einem mittleren Molekulargewicht zwischen 3500 und 7000 u. ä. Andere verwendbare Schutzkolloide sind z. B. Aminosäuren, wie Alanin, Pflanzengummis, wie Gummi arabicum usw. Die bevorzugte Menge an Schutzkolloid bzw. einer Mischung von Schutzkolloiden beträgt 20 bis 90 Gew.-%. Als besonders geeignet erwies sich eine Mischung von Zuckeralkohol und Polyalkylenglykol.

Das erfindungsgemäße Reagenz kann auch auf einem geeigneten Trägermaterial imprägniert vorliegen. In betracht kommt sowohl ein saugfähiges Trägermaterial, als auch ein quellfähiges, lösliches filmbildendes Trägermaterial. In dieser Form ermöglicht das erfindungsgemäße Reagenz die Herstellung von Teststreifen, welche direkt visuell oder mittels geeigneter Meßgeräte ausgewertet werden können.

Der erfindungsgemäße Farbtest zur Bestimmung der Phospholipasen liefert sehr genaue Ergebnisse bei hoher Empfindlichkeit. Er ist sehr einfach in der Handhabung und eignet sich sogar für Teststreifen. Da er nur eine sehr geringe oder gar keine lag-Phase aufweist, kann er auf die verschiedenen Analysenautomatensysteme ohne weiteres adaptiert werden.

Die Bestimmung selbst kann sowohl als Endpunktbestimmung als auch kinetisch durchgeführt werden. Gegenüber vielen bekannten Verfahren liegt in der kinetischen Durchführbarkeit der Vorteil, daß weder ein Abstoppen, noch ein Ausschütteln des gebildeten Reaktionsproduktes durchgeführt werden muß.

Die folgenden Beispiele erläutern die Erfindung weiter.

## Beispiel 1

1-O-Octadecyl-2-glutarsäure-p-nitrophenylester-sn-glycero-3-phosphocholin

a) 1-O-Octadecyl-2-glutarsäure-sn-glycero-3-phosphocholin

1 g 1-O-Octadecyl-sn-glycero-3-phosphocholin, 0,73 g Glutarsäureanhydrid, 0,2 g Dimethylaminopyridin werden in 30 ml Pyridin 70 h auf 50°C erhitzt. Dann wird das Lösungsmittel abgezogen und der Rückstand über LH 20 (Eluens: Chloroform/Methanol 1:1) chromatographiert. Anschließend wird über RP 18 chromatographiert (Laufmittel: Isopropanol/Wasser 9:1).
Ausbeute: 0,87 g
DC: $R_f$ = 0,26 (Kieselgel; Methanol)
Sprühreagenz: Hanes-Isherwood-Reagenz
$^1$H-NMR ($CDCl_3$):δ [ppm]: 0,87 (t,3H); 1,26 (m,32H); 1,90 (m,2H); 2,40 (m,4H); 3,1-3,6 (m,13H); 3,78 (m,2H); 4,02 (m,2H); 4,25 (m,2H); 5,18 (m,1H).

b) 1-O-Octadecyl-2-glutarsäure-p-nitrophenylester-sn-glycero-3-phosphocholin

310 mg 1a) werden in einem Gemisch aus Wasser/Tetrahydrofuran 1:1 gelöst und mit 70 mg p-Nitrophenol, 480 mg N-Ethyl-N'-dimethylaminopropylcarbodiimid versetzt. Anschließend wird 40 h bei 60°C gerührt. Nach Abdampfen des Lösungsmittels wird über RP 18 (Eluens: Isopropanol/Wasser 8:2) chromatographiert.
DC: $R_f$ = 0,21 (RP18; Isopropanol/Wasser 8:2)
$^1$H-NMR ($CDCl_3$):δ [ppm]: 0,88 (t,3H); 1,25 (m,32H); 2,04 (m,2H); 2,2-2,8 (m,4H);3,1-3,6 (m,13H); 3,80 (m,2H); 3,96 (m,2H); 4,28 (m,2H); 5,15 (m,1H); 7,30 (d,2H); 8,26 (d,2H).

Beispiel 2

1-O-Octadecyl-2-glutarsäure-methylresorufinester-sn-glycero-3-phosphocholin

Herstellung analog 1b) aus 31 mg 1a), 10 ml Wasser/Tetrahydrofuran 1:1, 110 mg 4 Methylresorufin, 48 mg N-Ethyl-N′-dimethylaminopropylcarbodiimid.
DC: $R_f$ = 0,20 (RP 18; Isopropanol/Wasser 8:2)
$^1$H NMR ($d_4$-Methanol):δ [ppm]: 0,90 (t,3H); 1,28 (m,32H); 1,92 (m,2H); 2,16 (s,3H); 2,44 (m,4H); 3,51 (m,2H); 3,69 (m,11H); 4,00 (m,2H); 4,29 (m,2H); 4,47 (m,2H); 5,2 (m,1H); 6,7-7,9 (m,5H).

Beispiel 3

1-O-Dodecyl-2-glutarsäure-p-nitrophenylester-glycero-3-sulfat

a) 1-O-Dodecyl-2-glutarsäure-3-O-trityl-glycerin

20,1 g 1-O-Dodecyl-3-O-trityl-glycerin, 9,2 g Glutarsäureanhydrid und 0,4 g Dimethylaminopyridin werden in 100 ml Pyridin 8 h auf 50°C erhitzt. Das Lösungsmittel wird abgezogen, der Rückstand in Essigester aufgenommen und mit 0,05 N Salzsäure geschüttelt. Nach Trocknung der Essigesterphase über Natriumsulfat wird das Lösungsmittel abgedampft und der Rückstand an Kieselgel (Laufmittel: Essigester/Petrolether 1:4) chromatographiert.
Ausbeute: 14 g
DC: $R_f$ = 0,20 (Kieselgel; Essigester/Petrolether 1:1)
$^1$H-NMR ($CDCl_3$):δ [ppm]: 0,88 (t,3H); 1,25 (m,20H); 1,99 (q,2H); 2,43 (t,4H); 3,15-3,45 (m,4H); 3,59 (d,2H); 5,22 (m,1H); 7,3 (m,15H).

b) 1-O-Dodecyl-2-glutarsäure-p-nitrophenylester-3-O-trityl-glycerin

8,6 g 3a) werden in 150 ml Chloroform gelöst und nacheinander 1,94 g p-Nitrophenol und 14,4 g Dicyclohexylcarbodiimid zugegeben. Nach 12-stündigem Rühren bei Raumtemperatur wird der Niederschlag abfiltriert und das Filtrat eingeengt. Der Rückstand wird an Kieselgel (Eluens: Essigester/Petrolether 1:4) chromatographiert.
Ausbeute: 8,8 g
DC: $R_f$ = 0,42 (Kieselgel; Essigester/Petrolether 1:4)
$^1$H-NMR ($CDCl_3$):δ [ppm]: 0,88 (t,3H); 1,25 (m,20H); 2,12 (q,2H); 2,53 (t,2H); 2,71 (t,2H); 3,2-3,5 (m,4H); 3,59 (d,2H); 5,28 (m,1H); 7,18 (d,2H); 7,30 (m,15H); 8,20 (d,2H).

c) 1-O-Dodecyl-2-glutarsäure-p-nitrophenylester-glycerin

6 g 3b) werden in Petrolether gelöst und auf mit Borsäure behandeltes Kieselgel aufgezogen. Das Produkt wird mit Petrolether/Essigester 8:2 heruntergewaschen.
Ausbeute: 2,7 g
DC: $R_f$ = 0,21 (Kieselgel; Chloroform/Aceton 49:1)
$^1$H-NMR ($CDCl_3$):δ [ppm]: 0,87 (t,3H); 1,25 (m,20H); 2,10 (m,2H); 2,53 (t,2H); 2,71 (t,2H); 3,45 (t,2H); 3,63 (d,2H); 3,82 (d,2H); 5,04 (m,1H); 7,28 (d,2H); 8,26 (d,2H).

d) 1-O-Dodecyl-2-glutarsäure-p-nitrophenylester-glycero-3-sulfat.

500 mg 3c) werden in 4 ml Chloroform aufgenommen und mit 0,22 ml Pyridin versetzt. Unter Eiskühlung wird 0,18 ml Chlorsulfonsäure in 2 ml Chloroform zugetropft. Dann wird die Reaktionslösung noch 2 h bei 0°C und 1 h bei Raumtemperatur gerührt. Nach Zugabe von 2 Tropfen Wasser wird eingeengt und der Rückstand in Chloroform aufgenommen. Nach Trocknung über Natriumsulfat wird das Lösungsmittel abgedampft und der Rückstand an Kieselgel (Laufmittel: Methylenchlorid/Methanol 8:1) chromatographiert.
Ausbeute: 180 mg
DC: $R_f$ = 0,26 (Kieselgel; Methylenchlorid/Methanol 6:1)
$^1$H-NMR ($CDCl_3$):δ [ppm]: 0,89 (t,3H); 1,21 (m;20H); 2,02 (m,2H); 2,54 (m,4H); 3,45 (m,4H); 4,19 (m,2H); 5,33 (m,1H); 7,28 (d,2H); 8,22 (d,2H).

Beispiel 4

1-O-Hexadecyl-2-glutarsäure-p-nitrophenylester-glycero-3-sulfat.

a) 1-O-Hexadecyl-2-glutarsäure-3-O-trityl-glycerin

Herstellung analog 3a) aus 15 g 1-O-Hexadecyl-3-O-trityl-glycerin, 50 ml Pyridin, 6,2 g Glutarsäureanhydrid, 0,4 g Dimethylaminopyridin.
Ausbeute: 7,14 g
DC: $R_f$ = 0,35 (Kieselgel; Essigester/Petrolether 2:3)
$^1$H-NMR ($CDCl_3$):δ [ppm]: 0,87 (t,3H); 1,1-1,65 (m,28H); 2,0 (m,2H); 2,43 (t,4H); 3,32 (m,4H); 3,59 (d,2H); 5,21 (m,1H); 7,33 (m,15H).

b) 1-O-Hexadecyl-2-glutarsäure-p-nitrophenylester-3-O-trityl-glycerin.

Herstellung analog 3b) aus 3,5 g 4a), 50 ml Chloroform, 0,84 g p-Nitrophenol, 6,18 g Dicyclohexylcarbodiimid.
Ausbeute: 2,85 g
DC: $R_f$ = 0,47 (Kieselgel; Essigester/Petrolether 1:4)
$^1$H-NMR ($CDCl_3$):δ [ppm]: 0,88 (t,3H); 1,25 (m,28H); 2,13 (q,2H); 2,53 (t,2H); 2,7 (t,2H); 3,33 (m,4H); 3,59 (d,2H); 5,28 (m,1H); 7,17 (d,2H); 7,33 (m,15H), 8,20 (d,2H).

c) 1-O-Hexadecyl-2-glutarsäure-p-nitrophenylester-glycerin

Herstellung analog 3c) aus 2,8 g 4b).
Ausbeute: 1,8 g
DC: $R_f$ = 0,22 (Kieselgel; Chloroform/Aceton 49:1)
$^1$H-NMR ($CDCl_3$):δ [ppm]: 0,87 (t,3H); 1,25 (m,28H); 2,12 (q,2H); 2,55 (m,4H); 3,35-4,0 (m,6H); 5,1 /m,1H); 7,28 (d,2H); 8,27 (d,2H).

d) 1-O-Hexadecyl-2-glutarsäure-p-nitrophenylester-glycero-3-sulfat.

Herstellung analog 3d) aus 500 mg 4c).
Ausbeute: 310 mg
DC: $R_f$ = 0,30 (Kieselgel; Methylenchlorid/Methanol 8:1)
$^1$H-NMR ($CDCl_3$):δ [ppm]: 0,87 (t,3H); 1,24 (m,28H); 2,03 (m,2H); 2,3-2,8 (m,4H); 3,20-3,70 (m,4H); 4,19 (m,2H); 5,31 (m,1H); 7,29 (d,2H); 8,23 (d,2H).

Beispiel 5

1-O-Dodecyl-2-adipinsäure-p-nitrophenylester-glycero-3-sulfat

a) 1-O-Dodecyl-2-O-benzyl-glycero-3-sulfat

28 g 1-O-Dodecyl-2-O-benzyl-glycerin werden in 200 ml Chloroform gelöst, 20 ml Pyridin zugegeben und bei 0°C tropfenweise eine Lösung von 11,2 ml Chlorsulfonsäure in 80 ml Chloroform zugefügt. Anschließend wird 3 h bei Raumtemperatur gerührt und dann das Gemisch auf Eis gegossen. Die Wasserphase wird 3 mal mit Chloroform geschüttelt und die organische Phase nach Trocknung über Natriumsulfat eingeengt. Der Rückstand wird an Kieselgel (Laufmittel: Chloroform/Methanol 4:1) chromatographiert.
Ausbeute: 23 g
DC: $R_f$ = 0,25 (Kieselgel; Methylenchlorid/Methanol 9:1)
Sprühreagenz: Dichlorfluorescein
$^1$H-NMR ($CDCl_3$):δ [ppm]: 0,88 (t,3H); 1,24 (m,20H); 3,15-3,50 (m,4H); 3,74 (m,1H); 4,0 (s,1H); 4,22 (m,2H); 4,59 (s,1H); 4,64 (s,1H); 7,26 (m,5H).

b) 1-O-Dodecyl-glycero-3-sulfat

22,8 g 5a) werden in 500 ml Methanol gelöst und an 2,3 g Pd/C hydriert. Der Reaktionsverlauf wird per

DC kontrolliert. Nach Ablauf der Reaktion wird filtriert und eingeengt. Der Rückstand wird in Aceton aufgeschlämmt und abfiltriert. Ausbeute: 15,6 g
DC: $R_f$ = 0,34 (Kieselgel; Methylenchlorid/Methanol 4:1)
$^1$H-NMR ($d_4$-Methanol):δ [ppm]: 0,89 (t,3H); 1,28 (m,20H); 3,4-3,6 (m,4H); 3,9-4,1 (m,3H).

c) p-Nitrophenyl-adipinsäure-anhydrid

1 g Adipinsäure-p-nitrophenylester werden 2 h in 40 ml Essigsäureanhydrid bei 80°C gerührt. Anschließend wird das Lösungsmittel abgezogen und der Rückstand am Hochvakuum getrocknet. Das Rohprodukt wird weiter umgesetzt.
DC: $R_f$ = 0,6 (Kieselgel; Essigester)

d) 1-O-Dodecyl-2-adipinsäure-p-nitrophenylester-glycero-3-sulfat.

0,44 g 5b), 5c) und 100 mg Dimethylaminopyridin werden in 50 ml Pyridin 5 h bei 80°C gerührt. Nach Abziehen des Lösungsmittels wird an Kieselgel (Laufmittel: Chloroform/Methanol 6:1) chromatographiert.
Ausbeute: 100 mg
DC: $R_f$ = 0,29 (Kieselgel; Chloroform/Methanol 6:1)
$^1$H-NMR ($CDCl_3$):δ [ppm]: 0,86 (t,3H); 1,23 (m,20H); 1,73 (m,4H); 2,20-2,75 (m,4H); 3,2-3,7 (m,4H); 4,16 (m,2H); 5,31 (m,1H); 7,29 (d,2H); 8,24 (d,2H).

Beispiel 6

Lösung 1:

125 mM Trispuffer pH 7,1, 4,0 mM Calciumchlorid, 250 µl/100 ml Triton X-100, 41 mg/100 ml Natriumdesoxycholat.

Lösung 2:

zu 1 ml der Lösung 1 werden 2 mg Substrat 1-O-Hexadecyl-2-glutarsäure-nitrophenylester-rac-glycero-3-sulfat unter leichtem Erwärmen bis zur Ausbildung einer Emulsion gerührt.
Hierzu werden 20 µl der Probe (Enzymlösung) gegeben. Der Verlauf der Reaktion wird photometrisch bei λ = 405 nm verfolgt.

Bei Auswertung über einen Standard bekannter Phospholipaseaktivität wird die Phospholipaseaktivität der Probe wie folgt berechnet:

$$\text{Aktivität}_{(\text{Probe})} = \frac{\text{Aktivität}_{(\text{Standard})} \cdot \Delta E/\text{min (Probe)}}{\Delta E/\text{min (Standard)}}$$

Eine Berechnung der Phospholipaseaktivität der Probe ist auch nach folgender Formel möglich:

$$\text{Aktivität (Probe) [U/l]} = 1000 \, \frac{V_{ges}}{\varepsilon \cdot V_{Probe} \cdot d} \cdot \Delta E/\text{min}$$

$V_{ges}$ : Gesamtvolumen des Testansatzes [$cm^3$]
$V_{Probe}$ : Volumen der Probe [$cm^3$]
ε : Extinktionskoeffizient des Chromogens bei 405 nm
d : Schichtdicke der Küvette [cm]
ΔE/min : Extinktionsänderung pro Minute bei 405 nm

Bei den genannten Reaktionsbedingungen beträgt der Extinktionskoeffizient $\varepsilon = 9{,}0 \cdot l \cdot cm^2 \cdot \mu mol^{-1}$.

## Patentansprüche

1. Phospholipasesubstrat der allgemeinen Formel

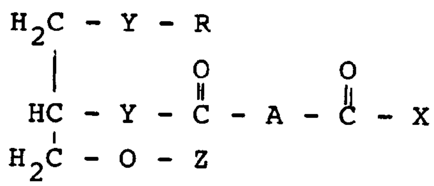

(I)

oder

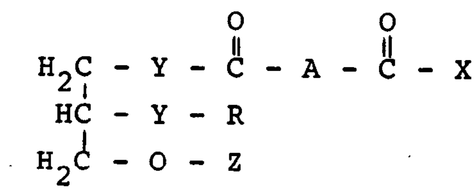

(II)

worin
A eine Alkylen- oder Alkenylengruppe mit 1 bis 16 C-Atomen,
R H oder eine Alkyl-, Alkenyl- oder Acylgruppe mit 1 bis 20 C-Atomen oder eine gegebenenfalls alkylsubstituierte Aryl- oder Aralkylgruppe mit 1 bis 8 C-Atomen in Alkylrest,
X den Rest einer aromatischen Hydroxy- oder Thiolverbindung und jedes Y unabhängig voneinander -S- oder -O-,
Z $-SO_3^{\ominus}$ oder

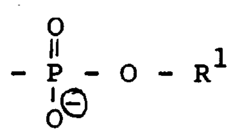

bedeuten, wobei $R^1$ sein kann ein Wasserstoffatom oder die Gruppe $(CH_2)_nNR^2_3$, in der n eine Zahl von 2 bis 4 und $R^2$ H oder $CH_3$ bedeuten,
oder Inositol,
oder Serin

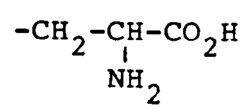

oder Glycerin.

2. Phospholipasesubstrat nach Anspruch 1,
**dadurch gekennzeichnet,**
daß R 6 bis 20 C-Atome aufweist.

3. Phospholipasesubstrat nach Anspruch 1 oder 2,
**dadurch gekennzeichnet,**
daß A 3 bis 7 C-Atome aufweist.

4. Phospholipasesubstrat nach Anspruch 1, 2 oder 3,
**dadurch gekennzeichnet,**
daß X ein gegebenenfalls substituierter Resorufin-, ein Chlorphenolrot-, Indoxyl-, Naphthol-, Thiophenol-, Thiofluoreszein- oder Phenolrest ist.

5. Verfahren zur optischen Bestimmung der Phospholipasen,
**dadurch gekennzeichnet,**
daß man ein Substrat gemäß Anspruch 1 der Einwirkung der phospholipasehaltigen Probe unterwirft und die Menge der freigesetzten aromatischen Hydroxy- oder Thiolverbindung direkt oder, nach Kopplung mit einem

geeigneten Chromogen die daraus gebildete Farbe, optisch bestimmt.

6. Verfahren nach Anspruch 5,
**dadurch gekennzeichnet,**
daß man die Bestimmung bei einer Calciumkonzentration im Test von 0,5 bis 10 mM/l vornimmt.

7. Reagenz zur optischen Bestimmung der Phospholipase,
**dadurch gekennzeichnet,**
daß es wenigstens eine Verbindung nach einem der Ansprüche 1 bis 4 und Puffersubstanz, sowie Gallensäure, $Ca^{++}$ und gegebenenfalls einen chromogenen Kuppler oder/und ein Hilfsenzym enthält.

8. Reagenz nach Anspruch 7,
**dadurch gekennzeichnet,**
daß es
0,05 - 10 mg/ml Substrat,
2 - 50 mg/ml Gallensäure,
0,5 - 10 mM/l $Ca^{++}$,
0 - 10 g/l Detergenz
20 - 250 mM/l Puffersubstanz,
jeweils bezogen auf gebrauchsfertige Lösung im Test enthält.

9. Reagenz nach Anspruch 7 oder 8,
**dadurch gekennzeichnet,**
daß es auf einem Trägermaterial imprägniert vorliegt.

## Claims

1. Phospholipase substrates of the general formula:-

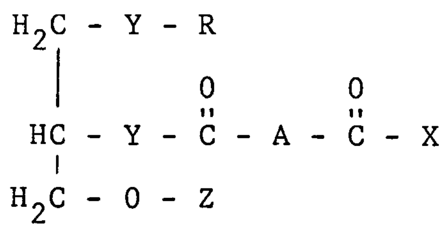

(I)

or

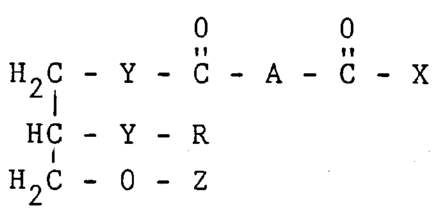

(II)

wherein
A signifies an alkylene or alkenylene group with 1 to 16 C-atoms,
R H or an alkyl, alkenyl or acyl group with 1 to 20 C-atoms or a possibly alkyl-substituted aryl or aralkyl group with 1 to 8 C-atoms in the alkyl radical,
X the residue of an aromatic hydroxy or thiol compound and each Y, independently of one another, -S- or -O-,
Z $-SO_3^{\ominus}$ or a radical of the general formula:-

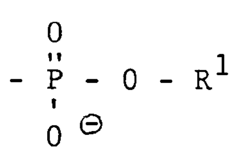

whereby $R^1$ can signify a hydrogen atom or the group $-(CH_2)_nR^2_3$, in which n signifies a number of 2 to 4 and $R^2$ H or $CH_3$,
or inositol
or serine

$$-CH_2-\underset{NH_2}{CH}-CO_2H$$

or glycerol.

2. Phospholipase substrates according to claim 1,
**characterised in that**
R has 6 to 20 C-atoms.

3. Phospholipase substrates according to claim 1 or 2,
**characterised in that**
A has 3 to 7 C-atoms.

4. Phospholipase substrates according to claim 1, 2 or 3,
**characterised in that**
X is a possibly substituted resorufin, a chlorophenol red, indoxyl, naphthol, thiophenol, thiofluorescein or phenol radical.

5. Process for the optical determination of phospholipases,
**characterised in that**
one subjects a substrate according to claim 1 to the action of a phospholipase- containing sample and optically determines the amount of the liberated aromatic hydroxy or thiol compound directly or, after coupling with a suitable chromogen, the colour formed therefrom.

6. Process according to claim 5,
**characterised in that**
one carries out the determination at a calcium concentration in the test of 0.5 to 10 mM/l.

7. Reagent for the optical determination of phospholipases,
**characterised in that**
it contains at least one compound according to one of claims 1 to 4 and a buffer substance, as well as bile acid, $Ca^{++}$ and possibly a chromogenic coupler and/or an auxiliary enzyme.

8. Reagent according to claim 7,
**characterised in that**
it contains
0,05 - 10 mg/ml of substrate,
2 - 50 mg/ml bile acid,
0,5 - 10 mM/l $Ca^{++}$
0 - 10 g/l detergent,
20 - 250 mM/l buffer substance,
in each case referred to the solution ready for use in the test.

9. Reagent according to claim 7 or 8,
**characterised in that**
it is present impregnated on a carrier material.

## Revendications

1. Substrat pour phospholipase de forme générale

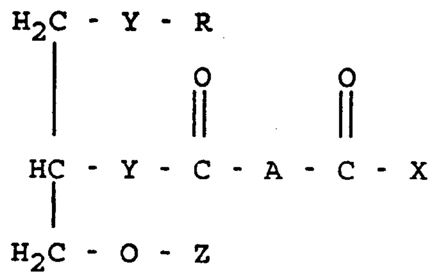 (I)

ou

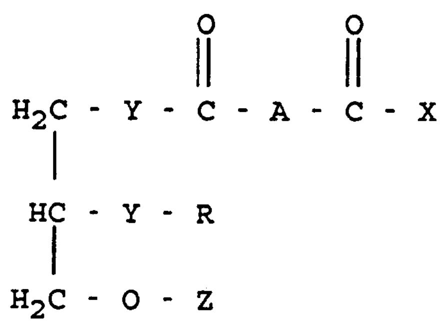 (II)

dans laquelle
A représente un groupe alkylène ou alcénylène de 1 à 16 atomes de carbone,
R représente H ou un groupe alkyle, alcényle ou acyle de 1 à 20 atomes de carbone ou un groupe aryle ou aralkyle éventuellement alkylsubstitué de 1 à 8 atomes de carbone dans le reste alkyle,
X représente le reste d'un composé hydroxy ou thiol aromatique et chaque Y représente indépendamment -S- ou -O-,
Z représente $-SO_3^{\ominus}$ ou

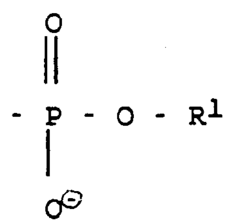

$R^1$ pouvant être un atome d'hydrogène ou le groupe $(CH_2)_nNR^2_3$ dans lequel n représente un nombre de 2 à 4 et $R^2$ représente H ou $CH_3$,
ou l'inositol,
ou la sérine

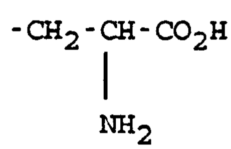

ou la glycérine.

2. Substrat pour phospholipase selon la revendication 1, **caractérisé en ce que** R comporte 6 à 20 atomes de carbone.

3. Substrat pour phospholipase selon la revendication 1 ou 2,
**caractérisé en ce que**
A comporte 3 à 7 atomes de carbone.

4. Substrat pour phospholipase selon la revendication 1, 2 ou 3,
**caractérisé en ce que**
X est un reste résorufine éventuellement substitué, un reste rouge de chlorophénol, indoxyle, naphtol, thiophénol, thiofluorescéine ou phénol.

5. Procédé de détermination optique des phospholipases,
**caractérisé en ce que**
l'on soumet un substrat selon la revendication 1 à l'action de l'échantillon contenant une phospholipase et l'on détermine optiquement la quantité de composé hydroxy ou thiol aromatique libéré directement ou l'on détermine la couleur formée après couplage avec un chromogène approprié.

6. Procédé selon la revendication 5,
**caractérisé en ce que**
l'on fait la détermination à une concentration en calcium dans le test de 0,5 à 10 mM/l.

7. Réactif pour la détermination optique de la phospholipase,
**caractérisé en ce qu'il**
contient au moins un composé selon l'une des revendications 1 à 4 et une substance tampon, ainsi que de l'acide biliaire, $Ca^{++}$ et éventuellement un coupleur chromogène et/ou une enzyme auxiliaire.

8. Réactif selon la revendication 7,
**caractérisé en ce qu'il**
contient
0,05 à 10 mg/ml de substrat,
2 à 50 mg/ml d'acide biliaire,
0,5 à 10 Mm/l de $Ca^{++}$,
0 à 10 g/l de détergent,
20 à 250 mM/l de substance tampon,
à chaque fois par rapport à la solution prête à l'emploi dans le test.

9. Réactif selon la revendication 7 ou 8,
**caractérisé en ce qu'il**
est présent sur un matériau support sous forme imprégnée.